# EUROPÄISCHE PATENTSCHRIFT

(11) **EP 0 439 816 B1**
(45) Veröffentlichungstag und Bekanntmachung des Hinweises auf die Patenterteilung: **01.09.1993**
(21) Anmeldenummer: 90125314.6
(22) Anmeldetag: 22.12.1990
(51) Int. Cl.: C07C 13/20, C07C 7/152

(54) **Verfahren zur Gewinnung von Cyclohexen aus solches enthaltenden Gemischen mit Benzol und/oder Cyclohexan**
Process for obtention of cyclohexene from mixtures containing cyclohexene, benzene and/or cyclohexane
Procédé pour l'obtention de cyclohexène à partir de mélanges contenant du cyclohexène, du benzène et/ou du cyclohexane

(30) Priorität: 31.01.1990 DE 4002764
(43) Veröffentlichungstag der Anmeldung: 07.08.1991
(73) Patentinhaber: BASF Aktiengesellschaft, 67063 Ludwigshafen (DE)
(72) Erfinder: Gosch, Hans-Jürgen, Dr., W-6702 Bad Duerkheim (DE); Fischer, Rolf, Dr., W-6900 Heidelberg (DE)

(56) Entgegenhaltungen:
- US-A- 2 406 645
- ANGEWANDTE CHEMIE, 98.Jg., Nr.8, 1968, Weinheim, E.WEBER et al. "Selektiver Einschluss und Trennung von isomeren und homologen Kohlewasserstoff-Wirtgitter" Seiten 719-721
- PATENT ABSTRACT OF JAPAN, unexamined applications, C Field, Band 4, Nr. 173, 29. 11. 1980 THE PATENT OFFICE JAPANESE GOVERNMENT Seite 69C32

## Beschreibung

Cyclohexen hat als Ausgangsverbindung zur Herstellung von Cyclohexanol besonderes Interesse erlangt. Geeignete Verfahren zur Herstellung von Cyclohexen sind z.B. die partielle Hydrierung von Benzol oder die Dehydrierung von Cyclohexan. Hierbei erhält man jedoch Gemische von Cyclohexen mit Benzol und/oder Cyclohexan. Eine Anreicherung oder Abtrennung des Cyclohexens aus solchen Gemischen erwies sich als sehr schwierig und aufwendig. Zumal die Siedepunkte von Cyclohexen, Cyclohexan und Benzol eng beieinander liegen und die Stoffe zudem miteinander Azeotrope bilden.

Entsprechend der EP-B 248 422 wurde deshalb auch schon versucht, Cyclohexen durch Azeotropdestillation aus den Gemischen mit Benzol und Cyclohexan abzutrennen. Bei einer solchen Azeotropdestillation ist es jedoch erforderlich, daß Benzol und Cyclohexan in einem bestimmten Mischungsverhältnis vorliegen. Dies hat den Nachteil, daß man nicht von Cyclohexen enthaltenden Gemischen beliebiger Zusammensetzung ausgehen kann.

Eine weitere Möglichkeit, Cyclohexen aus Gemischen mit Benzol und/oder Cyclohexan abzutrennen, bietet die Extraktivdestillation. Als geeignete Extraktionsmittel um die Flüchtigkeit von Cyclohexen, Benzol und/oder Cyclohexan zu beeinflussen, wurden bereits N-Methylpyrrolidon (JA 81 418), Adiponitril (JP-A 62/123 136), Phthalsäuredialkylester (JP-A 62/126 139) oder Dimethylacetamid (JP-A 58/164 524) vorgeschlagen. Die Abtrennung durch Extraktivdestillation ist jedoch energetisch sehr aufwendig und erfordert Rektifikationskolonnen mit sehr hoher Trennleistung. Nachteilig sind weiterhin die großen Mengen an Extraktionsmittel, die für diese Art der Trennung benötigt werden.

Es war deshalb die technische Aufgabe gestellt, ein Verfahren zur Abtrennung von Cyclohexen aus solches enthaltenden Gemischen mit Benzol und/oder Cyclohexan zu finden, das einfach durchführbar ist und energetisch weniger aufwendig ist und darüber hinaus keine großen Mengen an Lösungsmittel bedarf.

Diese Aufgabe wird gelöst in einem Verfahren zur Gewinnung von Cyclohexen aus solches enthaltenden Gemischen mit Benzol und/oder Cyclohexan, wobei man
a) Anthracen, das unter Reaktionsbedingungen inerte Substituenten haben kann, mit Cyclohexen im Gemisch mit Benzol und/oder Cyclohexan in flüssiger Phase umsetzt und ein Additionsprodukt aus Cyclohexen und Anthracen erhält,
b) das Additionsprodukt aus Cyclohexen und Antracen aus dem Reaktionsgemisch abtrennt und
c) das Additionsprodukt aus Cyclohexen und Antracen durch Erhitzen auf eine Temperatur von 150 bis 500°C in Anthracen und Cyclohexen zerlegt und Cyclohexen gewinnt.

Das neue Verfahren hat den Vorteil, daß man von Cyclohexen enthaltenden Gemischen ausgeht, die bei der Hydrierung von Benzol oder der Dehydrierung von Cyclohexan direkt anfallen. Weiter hat das neue Verfahren den Vorteil, daß es wenig aufwendig ist und keine großen Mengen an Extraktionsmittel im Kreis geführt werden müssen.

Aus der US-Patentschrift 2 406 645 ist zwar schon bekannt, daß man durch Erhitzen von Antracen oder dessen Derivaten mit Cyclohexen Additionsprodukte aus Antracen und Cyclohexen erhält. Es wird jedoch kein Hinweis gegeben, wie die gestellte technische Aufgabe zu lösen ist.

Erfindungsgemäß geht man von Gemischen aus, die neben Cyclohexen Benzol und/oder Cyclohexan enthalten. Bevorzugte Gemische enthalten neben Cyclohexan und/oder Benzol 5 bis 50 Gew.-% Cyclohexen. Ein typisches Ausgangsgemisch enthält beispielsweise 10 bis 40 Gew.-% Cyclohexen, 1 bis 50 Gew.-% Cyclohexan und 10 bis 90 Gew.-% Benzol.

In der Stufe a wird das im Gemisch mit Benzol und/oder Cyclohexan vorliegende Cyclohexen mit Anthracen, das unter Reaktionsbedingungen inerte Substituenten haben kann, in flüssiger Phase umgesetzt und ein Additionsprodukt aus Cyclohexen und Antracen erhalten.

Neben Anthracen sind auch substituierte Anthracene geeignet, die Alkylgruppen mit 1 bis 8 Kohlenstoffatomen, Cycloalkylgruppen mit 5 bis 8 Kohlenstoffatomen, Aralkylgruppen mit 7 bis 8 Kohlenstoffatomen, Phenylgruppen, OH-Gruppen, Alkoxygruppen mit 1 bis 4 Kohlenstoffatomen, Carbalkoxygruppen mit 2 bis 5 Kohlenstoffatomen, Nitrilgruppen, Nitrogruppen, Carboxylgruppen, Carbonsäureanhydridgruppen, Formylgruppen, Carbonsäurehalogenidgruppen oder Halogenatome als Substituenten haben können. Solche substituierten Antracene enthalten in der Regel 1 bis 4 Substituenten, insbesondere Substituenten, die aus Kohlenstoffatomen, Wasserstoffatomen und gegebenenfalls Sauerstoffatomen aufgebaut sind.

Vorteilhaft verwendet man neben Anthracen substituierte Antracene, die 1 oder 2 Alkyl- oder Phenylgruppen der vorgenannten Kohlenstoffzahl haben. Besondere Bedeutung haben Alkylanthracene erlangt. Im folgenden seien unter der Bezeichnung Anthracen jeweils dessen vorgenannte Derivate miteingeschlossen.

Geeignete Anthracenderivate sind beispielsweise 9-Methylanthracen, 9,10-Dimethylanthracen, 9-Chloranthracen, 9-Carbonmethoxyanthracen, 9-Ethylanthracen, 9-Phenylanthracen, 9,10-Dichloranthracen, 9-Anthracencarbonsäurenitril, 9-Formylanthracen oder 2-Methylanthracen.

Im allgemeinen verwendet man je Mol Cyclohexen 0,1 bis 50 mol Anthracen vorteilhaft 1 bis 30 mol, insbesondere im Überschuß z.B. von 1,2 bis 10 mol Antracen.

Die Umsetzung führt man im allgemeinen bei einer Temperatur von 20 bis 400°C vorteilhaft von 50 bis 300°C durch. Besonders bewährt hat sich eine Temperatur von 100 bis 300°C. Bei der Umsetzung wendet man im allgemeinen von Atmosphärendruck, verminderten oder erhöhten Druck an. Vorteilhaft führt man die Umsetzung unter erhöhtem Druck z.B. von 10 bis 300 bar durch. Die Druck- und Temperaturbedingungen werden so gewählt, daß die Umsetzung in flüssiger Phase durchgeführt wird.

Im allgemeinen hält man eine Reaktionszeit von 0,1 bis 100 Std. und vorteilhaft 0,1 bis 20 Std., insbesondere 0,1 bis 10 Std., ein. Die Umsetzung kann einstufig oder mehrstufig kontinuierlich oder diskontinuierlich ohne Mitverwendung von Katalysatoren durchgeführt werden.

In der Stufe b wird das Additionsprodukt aus Cyclohexen und Anthracen im folgenden Addukt genannt, aus dem Reaktionsgemisch abgetrennt. Das Reaktionsgemisch besteht im wesentlichen aus Addukt Benzol und/oder Cyclohexan ggf. nicht umgesetztem Cyclohexen und überschüssigem Anthracen. Nach einer Arbeitsweise erhält man das Addukt beispielsweise durch Kristallisation und Abtrennen durch Filtration. Nach einer bevorzugten Arbeitsweise werden Benzol und/oder Cyclohexan abdestilliert und Addukt ggf. im Gemisch mit nichtumgesetztem Anthracen erhalten. Das Addukt kann durch Kristallisation z.B. aus Alkanolen gereinigt werden.

Nicht umgesetztes Cyclohexen enthaltende Gemische werden zweckmäßig in die Stufe a zurückgeführt.

In der Stufe c wird das Additionsprodukt aus Cyclohexen und Anthracen durch Erhitzen auf eine Temperatur von 150 bis 500°C in Anthracen und Cyclohexen zerlegt und Cyclohexen gewonnen.

Vorteilhaft hält man eine Temperatur von 200 bis 400°C ein. Im allgemeinen führt man die Zersetzung bei Atmosphärendruck, vermindertem Druck oder erhöhtem Druck durch, z.B. von 0,01 bis 100 bar, vorteilhaft 0,1 bis 10 bar. Vorzugsweise wird die Zerlegung des Addukts in der Gasphase z.B. unter Mitverwendung eines Inertgasstromes wie Stickstoff oder Argon durchgeführt. Anthracen und Cyclohexen gewinnt man dann aus der Gasphase durch Abkühlen und Kondensieren. Das so erhaltene Gemisch aus Cyclohexen und Anthracen läßt sich dann auf einfache Weise z.B. durch Destillation oder Filtration trennen.

Die Spaltung des Addukts kann ein- oder mehrstufig, kontinuierlich oder diskontinuierlich durchgeführt werden.

Das zurückgewonnen Anthracen und ggf. nicht umgesetztes Addukt werden zweckmäßig in die Stufe a zurückgeführt.

Das nach dem Verfahren der Erfindung erhältliche Cyclohexen eignet sich zur Herstellung von Cyclohexanol, einem wichtigen Ausgangsstoff zur Herstellung von Faserrohstoffen.

Das Verfahren nach der Erfindung sei an folgenden Beispielen veranschaulicht.

### Beispiele

### Beispiel 1

a) In einem 300 ml Rührautoklaven wurde eine Mischung aus 24 g (0,29 Mol) Cyclohexen, 36 g (0,46 Mol) Benzol und 60 g (0,71 Mol) Cyclohexan mit 63 g (0,35 Mol) Anthracen versetzt und der Autoklav mit Stickstoff auf einen Druck von 10 bar gebracht. Anschließend wurde das Gemisch 8 h auf 250°C erwärmt. Der Druck betrug dabei etwa 40 bar.
b) Nach Abkühlen und Entspannen wurde das Reaktionsgemisch in eine Destillationsapparatur gegeben und Benzol, Cyclohexan und nicht umgesetztes Cyclohexen vom Cyclohexen-Anthracen-Addukt und überschüssigen Anthracen getrennt. Es wurde eine Gemisch aus 18,1 g (0,22 Mol) Cyclohexen, 36 g (0,46 Mol) Benzol und 60 g (0,71 Mol) Cyclohexan und ein Gemisch aus 18,2 g (0,07 Mol) Addukt und 50,3 g (0,28 Mol) Anthracen erhalten. Die Zusammensetzung der Mischungen wurde durch Gaschromatographie bestimmt. Der Umsatz betrug somit 24,1 Mol-%, bezogen auf das eingesetzte Cyclohexen.
c) Das Cyclohexen-Anthracen-Addukt wurde durch Umkristallisieren aus Toluol und Ethanol auf 80 Gew.-% angereichert. 2,02 g dieses Gemisches (80 Gew.-% Addukt, 0,0062 Mol) wurden in einem Quarzrohr im leichten Argonstrom bei 1 bar für 10 Minuten auf 360°C erwärmt. Das Gemisch aus Addukt und Anthracen verdampfte dabei vollständig. Die Reaktionsprodukte der Thermolyse wurden in zwei hintereinander geschalteten, gut gekühlten Kühlfallen aufgefangen. Die gaschromatographische Analyse des Inhaltes der Kühlfallen ergab folgendes Ergebnis:
   0,46 g (0,0056 Mol) Cyclohexen
   0,156 g (0,0006 Mol) Addukt
   1,4 g (0,00786 Mol) Anthracen
   Der Umsatz des Adduktes zu Cyclohexen und Anthracen betrug somit 90,3 Mol-%.

### Beispiel 2

Wie im Beispiel 1 beschrieben, wurde ein Gemisch aus 35 g (0,43 Mol) Cyclohexen, 30 g (0,38 Mol) Benzol, 50 g (0,59 Mol) Cyclohexan und 91,2 g (0,51 Mol) Anthracen 8 h auf 250°C erhitzt. Der Austrag bestand aus 50 g (0,59 Mol) Cyclohexan, 30 g (0,38 Mol) Benzol, 26,3 g (0,32 Mol) Cyclohexen, 27,6 g (0,10 Mol) Addukt und 72,3 g (0,4 Mol) Anthracen. Der Umsatz betrug somit 23,2 Mol-%, bezogen auf das eingesetzte Cyclohexen.

Benzol, Cyclohexan und nicht umgesetztes Cyclohexen wurden abdestilliert.

1,63 g des erhaltenen Gemisches aus Cyclohexen-Anthracen-Addukt und Anthracen (27,6 Gew.-% Addukt im Gemisch, 0,00173 Mol Addukt) wurden in einem Quarzrohr im leichten Argonstrom 15 Minuten auf 320°C erwärmt. Die Reaktionsprodukte wurden in zwei Kühlfallen kondensiert und anschließend analysiert. Es wurde folgendes Ergebnis erhalten:
0,122 g (0,00149 Mol) Cyclohexen
0,063 g (0,00024 Mol) Addukt
1,446 g (0,008 Mol) Anthracen

Der Umsatz des Adduktes zu Cyclohexen und Anthracen betrug somit 86,1 Mol-%.

### Beispiel 3

Wie im Beispiel 1 beschrieben, wurde ein Gemisch aus 40 g (0,49 Mol) Cyclohexen, 15 g (0,19 Mol) Benzol, 25 g (0,3 Mol) Cyclohexan und 104,2 g (0,58 Mol) Anthracen für 8 h auf 250°C erwärmt. Der Austrag bestand aus 15 g (0,19 Mol) Benzol, 25 g (0,3 Mol) Cyclohexan, 26,7 g (0,33 Mol) Cyclohexen, 42,2 g (0,16 Mol) Addukt und 75,3 g (0,42 Mol) Anthracen. Der Umsatz betrug somit 33 Mol-%, bezogen auf das eingesetzte Cyclohexen.

### Beispiel 4

Wie in Beispiel 1 beschrieben, wurde ein Gemisch aus 24 g (0,29 Mol) Cyclohexen, 36 g (0,46 Mol) Benzol, 60 g (0,71 Mol) Cyclohexan und 62,6 g (0,35 Mol) Anthracen für 4 h auf 270°C erwärmt. Der Austrag bestand aus 60 g (0,71 Mol) Cyclohexan, 36 g (0,46 Mol Benzol, 20,4 g (0,25 Mol) Cyclohexen, 11,4 g (0,044 Mol) Addukt und 54,8 g (0,31 Mol) Anthracen. Der Umsatz betrug somit 15 Mol-%, bezogen auf das eingesetzte Cyclohexen.

### Beispiel 5

Wie im Beispiel 1 beschrieben, wurde ein Gemisch aus 8 g (0,097 Mol) Cyclohexen, 12 g (0,15 Mol) Benzol, 20 g (0,23 Mol) Cyclohexan und 104,3 g (0,59 Mol) Anthracen für 8 h auf 250°C erwärmt. Der Austrag bestand aus 12 g (0,15 Mol) Benzol, 20 g (0,23 mol) Cyclohexan, 4,5 g (0,055 Mol) Cyclohexen, 11,1 g (0,043 Mol) Addukt und 96,7 g (0,543 Mol) Anthracen. Der Umsatz betrug somit 44,3 Mol-%, bezogen auf das eingesetzte Cyclohexen.

### Beispiel 6

Ein Gemisch aus 0,44 g (0,0054 Mol) Cyclohexen und 1,15 g (0,006 Mol) 9-Methylanthracen wurde in einem Autoklaven für 7 h unter einem Druck von ca. 40 bar auf 250°C erwärmt. Der Reaktionsaustrag bestand aus 0,837 g (0,0028 Mol) Addukt, 0,28 g (0,0026 Mol) Cyclohexen und 0,603 g (0,0032 Mol) 9-Methylanthracen. Der Umsatz betrug somit 52 Mol-%, bezogen auf das eingesetzte Cyclohexen.

### Beispiel 7

Ein Gemisch aus 0,41 g (0,005 Mol) Cyclohexen und 1,53 g (0,006 Mol) 9-Phenylanthracen wurde in einem Autoklaven für 7 h unter einem Druck von ca. 40 bar auf 250°C erwärmt. Der Reaktionsaustrag bestand aus 0,57 g (0,00158 Mol) Addukt, 0,28 g (0,0034 Mol) Cyclohexen und 1,09 g (0,0043 Mol) 9-Phenylanthracen. Der Umsatz betrug somit 32 Mol-%, bezogen auf das eingesetzte Cyclohexen.

## Patentansprüche

1. Verfahren zur Gewinnung von Cyclohexen aus solches enthaltenden Gemischen mit Benzol und/oder Cyclohexan, dadurch gekennzeichnet, daß man
a) Anthracen, das unter Reaktionsbedingungen inerte Substituenten enthalten kann, mit Cyclohexen im Gemisch mit Benzol und/oder Cyclohexan in flüssiger Phase umsetzt und ein Additionsprodukt aus Cyclohexen und Anthracen erhält,
b) das Additionsprodukt aus Cyclohexen und Anthracen aus dem Reaktionsgemisch abtrennt und
c) das Additionsprodukt aus Cyclohexen und Anthracen durch Erhitzen auf eine Temperatur von 150 bis 500°C in Anthracen und Cyclohexen zerlegt und Cyclohexen gewinnt.

2. Verfahren nach Anspruch 1, dadurch gekennzeichnet, daß man je Mol Cyclohexen 1 bis 30 Mol Anthracen anwendet.

3. Verfahren nach den Ansprüchen 1 und 2, dadurch gekennzeichnet, daß man in der Stufe a eine Temperatur von 100 bis 300°C einhält.

4. Verfahren nach den Ansprüchen 1 bis 3, dadurch gekennzeichnet, daß man in der Stufe a einen Druck von 10 bis 300 bar einhält.

5. Verfahren nach den Ansprüchen 1 bis 4, dadurch gekennzeichnet, daß man Anthracen, das bis zu 2 Alkylgruppen mit 1 bis 4 Kohlenstoffatomen als Substituenten hat, verwendet.

6. Verfahren nach den Ansprüchen 1 bis 5, dadurch gekennzeichnet, daß man in der Stufe c eine Temperatur von 200 bis 400°C einhält.

## Claims

1. A process for isolating cyclohexene from mixtures thereof with benzene and/or cyclohexane, which comprises
a) reacting, in the liquid phase, anthracene, which may contain substituents inert under the reaction conditions, with cyclohexene in the mixture with benzene and/or cyclohexane, giving an addition product of cyclohexene and anthracene,
b) separating off the addition product of cyclohexene and anthracene from the reaction mixture and
c) decomposing to give the addition product of cyclohexene and anthracene anthracene and cyclohexene by heating to from 150 to 500°C and isolating cyclohexene.

2. A process as claimed in claim 1, wherein from 1 to 30 mol of anthracene are used per mole of cyclohexene.

3. A process as claimed in claims 1 and 2, wherein a temperature of from 100 to 300°C is maintained in stage a.

4. A process as claimed in any of claims 1 to 3, wherein a pressure of from 10 to 300 bar is maintained in stage a.

5. A process as claimed in any of claims 1 to 4, wherein anthracene containing up to 2 alkyl groups having from 1 to 4 carbon atoms as substituents is used.

6. A process as claimed in any of claims 1 to 5, wherein a temperature of from 200 to 400°C is maintained in stage c.

## Revendications

1. Procédé d'obtention du cyclohexène à partir de mélanges contenant ce dernier et de benzène et/ou de cyclohexane, caractérisé en ce que
a) on fait réagir l'anthracène, qui peut contenir des substituants inertes dans les conditions réactionnelles, avec le cyclohexène en mélange au benzène et/ou au cyclohexane en phase liquide et on obtient ainsi un produit d'addition du cyclohexène et de l'anthracène,
b) on sépare le produit d'addition du cyclohexène et de l'anthracène du mélange réactionnel et
c) on décompose le produit d'addition du cyclohexène et de l'anthracène en anthracène et cyclohexène par chauffage jusqu'à une température de 150 à 500°C et on récupère le cyclohexène.

2. Procédé suivant la revendication 1, caractérisé en ce que l'on utilise de 1 à 30 moles d'anthracène par mole de cyclohexène.

3. Procédé suivant les revendications 1 et 2, caractérisé en ce que l'on maintient une température de 100 à 300°C au cours de la mise en oeuvre de l'étape a.

4. Procédé suivant l'une quelconque des revendications 1 à 3, caractérisé en ce que l'on maintient une pression de 10 à 300 bars au cours de la mise en oeuvre de l'étape a.

5. Procédé suivant l'une quelconque des revendications 1 à 4, caractérisé en ce que l'on utilise de l'anthracène qui comporte jusqu'à deux radicaux alkyle avec de 1 à 4 atomes de carbone à titre de substituants.

6. Procédé suivant les revendications 1 à 5, caractérisé en ce que l'on maintient une température de 200 à 400°C au cours de la mise en oeuvre de l'étape c.
